# FASCICULE DE BREVET EUROPEEN

(11) **EP 4 446 720 B1**
(45) Date de publication et mention de la délivrance du brevet: **25.11.2026**
(21) Numéro de dépôt: 24168480.2
(22) Date de dépôt: 04.04.2024
(51) Int. Cl.: G01N 1/04, C12Q 1/04, G01N 1/28, G01N 33/569, G01N 1/02, B01F 31/10, B01F 31/24, B01F 31/25, B01F 33/25

(54) **SYSTÈME ET PROCÉDÉ DE PRÉPARATION D'ÉCHANTILLONS BIOLOGIQUES**
SYSTEM UND VERFAHREN ZUR AUFBEREITUNG BIOLOGISCHER PROBEN
SYSTEM AND METHOD FOR PREPARING BIOLOGICAL SAMPLES

(30) Priorité: 12.04.2023 FR 2303626
(43) Date de publication de la demande: 16.10.2024
(73) Titulaire: Labo Moderne, 92230 Gennevilliers (FR)
(72) Inventeur: REFET, Alain, GENNEVILLIERS (FR); CHARLET, Jean-Baptiste, GENNEVILLIERS (FR)
(74) Mandataire: Cabinet Beau de Loménie

(56) Documents cités:
- US-A- 5 921 477
- US-A1- 2009 004 688
- US-A1- 2020 150 005
- HOLINKA JOHANNES ET AL: "Sonication cultures of explanted components as an add-on test to routinely conducted microbiological diagnostics improve pathogen detection", vol. 29, no. 4, 7 April 2010 (2010-04-07), US, pages 617 - 622, XP093095333, ISSN: 0736-0266, Retrieved from the Internet <URL:https://api.wiley.com/onlinelibrary/tdm/v1/articles/10.1002%2Fjor.21286> [retrieved on 20231026], DOI: 10.1002/jor.21286

## Description

### Domaine Technique

La présente invention concerne le domaine des analyses d'échantillons, et plus précisément la préparation d'échantillons biologiques en vue de telles analyses, notamment bactériologiques, par exemple dans le domaine médical.

### Technique antérieure

La préparation d'échantillons est une opération consistant à préparer un échantillon en vue d'effectuer une analyse.

Des procédés et des dispositifs de préparation d'échantillons sont par exemple présentés dans US 2009/004688 A1, US 5 921 477 A, US 2020/150005 A1 et Holinka J, Bauer L, Hirschl AM, Graninger W, Windhager R, Presterl E. Sonication cultures of explanted components as an add-on test to routinely conducted microbiological diagnostics improve pathogen detection. J Orthop Res. 2011 Apr;29(4):617-22. doi: 10.1002/jor.21286. Epub 2010 Dec 7. PMID: 21337398.

Dans une grande majorité de cas, les échantillons biologiques sont préparés préalablement à l'analyse, l'échantillon tel que prélevé ne pouvant pas être analysé directement de manière satisfaisante.

La préparation consiste à mettre l'échantillon en forme, le traiter mécaniquement et/ou chimiquement de manière à l'adapter à la technique d'analyse choisie.

Les méthodes d'analyses reposent communément sur des analyses statistiques, ou itératives, basées sur une série d'analyses à partir de sous-échantillons obtenus par préparation d'un même échantillon. Pour ce faire, on comprend donc bien qu'il est nécessaire de disposer de plusieurs échantillons d'analyse identiques.

La préparation doit donc répondre à une condition d'homogénéité afin que chaque analyse réalisée sur un sous-échantillon soit représentative de l'échantillon dont il est issu.

Il est également nécessaire que la préparation soit réalisée dans un milieu stérile et étanche, afin de ne pas introduire d'éléments étrangers dans l'échantillon.

Plusieurs techniques de préparation sont connues, en fonction du type d'analyse à effectuer et de la nature de l'échantillon, notamment si l'échantillon est couplé à de la matière solide, par exemple de l'os ou un élément orthopédique tel qu'une vis, et qu'il est alors nécessaire de récurer la matière solide pour en extraire l'échantillon.

On connait ainsi les techniques de préparation par broyage mécanique au moyen d'un outil en rotation autour d'un axe entrant en contact direct avec la matière, mais de telles techniques nécessitent une stérilisation et un nettoyage systématique de l'outil entre deux préparations afin de ne pas contaminer les échantillons.

D'autres techniques ont également été proposées, dans lesquelles un élément d'agitation est intégré dans un récipient. Le récipient est maintenu fixe, et l'élément d'agitation est actionné de manière à entrainer des éléments tels que des billes en mouvement, qui entrent alors en contact avec la matière pour récurer l'échantillon quand nécessaire, fragmenter la matière et l'homogénéiser. Une telle technique est intéressante en ce qu'elle utilise des récipients à usage unique ce qui est avantageux en ce qu'elle remédie à la problématique de nettoyage des outils évoquée précédemment.

En revanche, l'intégration de l'élément d'agitation dans le récipient pose des problématiques importantes en termes d'étanchéité. Les dispositifs actuels dans lesquels l'entraînement mécanique de l'élément d'agitation se fait à partir de l'extérieur du récipient, il est présent obligatoirement un couplage mécanique entre l'extérieur et l'intérieur du récipient. Ce couplage peut conduire à des fuites, ce qui est très problématique pour plusieurs raisons. Les fuites de liquide peuvent endommager l'appareil électrique servant à actionner l'élément d'agitation. De plus, la fuite d'éléments contaminés peut entrainer un risque de contamination de l'utilisateur du dispositif, ce qui n'est évidemment pas acceptable.

En outre, ces techniques utilisent des récipients faits de plusieurs plastiques différents, ce qui rend d'autant plus complexe leur stérilisation dans la mesure où le procédé doit également être adapté au récipient afin de ne pas l'endommager.

Enfin, ces techniques nécessitent une opération supplémentaire de récurage de matière solide, introduisant des difficultés supplémentaires quant au respect des conditions de stérilité et d'étanchéité du milieu, ainsi que relativement à l'homogénéité du sous-échantillon prélevé par rapport à l'échantillon.

Un besoin existe pour une technique permettant l'extraction et la préparation d'un échantillon biologique qui soit dépourvue, au moins partiellement, des inconvénients précités, tout en permettant ensuite de réaliser une culture de l'échantillon biologique.

### Exposé de l'invention

La présente invention vise ainsi à répondre au moins partiellement à ces problématiques.

A cet effet, la présente invention concerne un procédé de préparation d'échantillons biologiques pour une analyse bactériologique tel que défini en revendication 1, dans lequel- on dispose un support portant un échantillon biologique dans un conteneur contenant des billes,
- on obture le conteneur de manière étanche,
- on agite le conteneur dans lequel sont disposés l'échantillon, les billes, de manière à mettre en mouvement les billes et ainsi provoquer des chocs sur le support de manière à séparer l'échantillon du support.

L'échantillon biologique peut par exemple être un échantillon biologique d'un sujet présentant des symptômes cliniques et/ou soupçonné de présenter une infection.

Typiquement, on prélève et on dispose un résidu de l'échantillon dans un milieu de culture, optionnellement un milieu de culture sélectif.

Typiquement, on caractérise la présence ou l'absence de microorganismes pathogènes dans le résidu d'échantillon.

Typiquement, on agite le conteneur selon un mouvement non rotatif. Par exemple, le conteneur est agité selon un mouvement non rotatif curviligne.

Le mouvement peut comprendre au moins une combinaison de mouvements parmi les combinaisons suivantes :
- une combinaison de translations alternatives ayant des directions successives deux à deux formant un angle supérieur à 10° ; et
- une combinaison d'au moins une translation et d'au moins une rotation.

Typiquement, préalablement à l'obturation du conteneur, on ajoute un liquide dans le conteneur.

Typiquement, l'échantillon est un biofilm.

Un aspect qui n'est pas revendiqué en tant que tel concerne un système pour la préparation d'échantillons biologiques, comprenant
- un conteneur muni d'un couvercle adapté pour obturer de manière étanche le conteneur, le conteneur étant adapté pour recevoir un échantillon biologique et comprenant des billes adaptées pour agir sur ledit échantillon,
- un secoueur, caractérisé en ce que le secoueur est adapté pour entrainer en mouvement le conteneur de manière à mettre en mouvement les billes et ainsi provoquer des chocs sur le support de manière à séparer l'échantillon du support.

Par « secoueur », on désigne généralement un dispositif adapté pour entrainer un élément, ici le conteneur, en mouvement. Le secoueur selon l'invention est adapté de manière à entrainer en mouvement le contenu du conteneur afin de générer des chocs entre les différents éléments du contenu, en particulier entre les billes et l'échantillon.

Les billes ont typiquement un diamètre compris entre 10 mm et 50 mm.

Le secoueur est avantageusement adapté pour entrainer le conteneur selon un mouvement non rotatif. Par exemple, le secoueur est adapté pour entrainer le conteneur selon un mouvement non rotatif curviligne.

Le conteneur comprend typiquement un liquide. Par exemple, le liquide est un liquide de conservation.

Le conteneur comprend typiquement un ou plusieurs sous-ensembles de billes comprenant chacun au moins une bille d'un diamètre déterminé en un même matériau.

### Brève description des dessins

D'autres caractéristiques, buts et avantages de l'invention ressortiront de la description qui suit, qui est purement illustrative et non limitative, et qui doit être lue en regard des dessins annexés, sur lesquels :
[Fig. 1][Fig. 2] Les figures 1 et 2 présentent un exemple de conteneur et un système permettant de mettre en œuvre un procédé selon un aspect de l'invention,
[Fig. 3] La figure 3 représente schématiquement un procédé selon un aspect de l'invention.

Sur l'ensemble des figures, les éléments en commun sont repérés par des références numériques identiques.

### Description des modes de réalisation

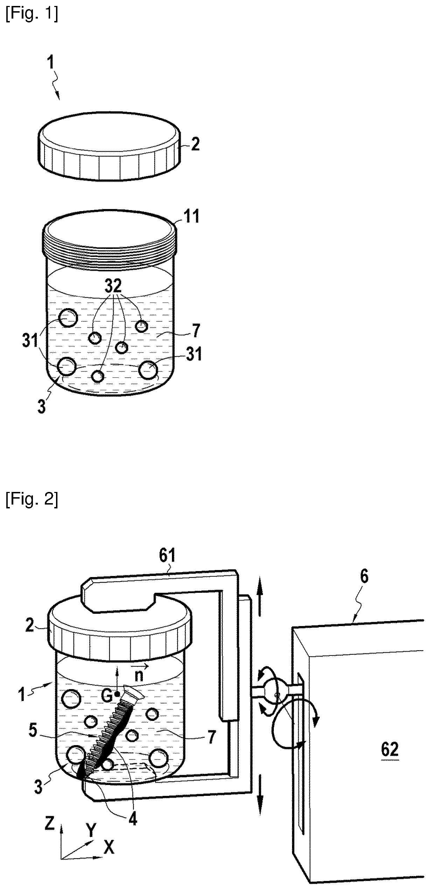

Les figures 1 et 2 présentent un exemple de conteneur et un système permettant de mettre en œuvre un procédé selon un aspect de l'invention.

La figure 1 représente un conteneur 1 de forme cylindrique, présentant une extrémité libre 11 munie d'un filetage adapté pour permettre qu'un couvercle 2 obture de manière étanche le conteneur 1, par exemple par vissage.

Le conteneur 1 a typiquement un volume compris entre 10ml et 1000 ml, voire entre 100ml et 750ml, voire entre 150ml et 500ml.

De tels volumes de conteneur 1 permettent notamment d'y introduire un échantillon biologique 4 porté par un support 5 et de fournir un espace libre suffisant à la circulation des billes 3 pour réaliser les opérations de récurer la matière biologique, la fragmenter et l'homogénéiser. Le support 5 peut être un support biologique et/ou non biologique. Par exemple, le support 5 peut contenir de la matière osseuse, ou bien une vis à os, typiquement une vis à os métallique, un implant ou une prothèse.

Le conteneur 1 peut présenter différentes formes.

Il peut s'agir d'un conteneur cylindrique de révolution tel que représenté sur les figures, d'un tube, d'un conteneur ayant une forme générale cylindrique ou cylindro-conique, d'une fiole.

Le couvercle 2 peut être réalisé de différentes manières ; par exemple couvercle tel qu'illustré, un capuchon ou une membrane étanche, dès lors que la fonction d'obturation étanche est réalisée.

Le conteneur 1 comprend des billes 3, et est adapté pour recevoir un échantillon biologique 4.

Le conteneur 1 comprend également un liquide 7, pouvant être un liquide de conservation dans le cas d'échantillons biologiques solides ou semi-solides tels que des prélèvements de tissus, ou être un liquide spécifique à l'échantillon à analyser.

Dans le mode de réalisation représenté sur les figures 1 et 2, l'échantillon biologique 4 se trouve sur un support 5, en l'occurrence une vis, par exemple une vis à os.

Dans l'exemple représenté sur les figures, les billes 3 comprennent deux sous-ensembles de billes que l'on désigne par 31 et 32, comprenant chacun un nombre donné de billes de diamètres distincts.

Dans l'exemple présenté, le sous ensemble 31 comprend 3 billes d'un diamètre D1, et le sous ensemble 32 comprend 4 billes d'un diamètre D2 < D1.

Le nombre total de billes 3 peut être compris entre 1 et 1000 ; ou entre 5 et 500, ou entre 5 et 250, ou encore entre 5 et 50, ou encore entre 5 et 20, ou par exemple égal à 10.

Ces nombres totaux de billes 3 sont compatibles avec une bonne cinétique des opérations de récurer le support 5, éventuellement le fragmenter et l'homogénéiser de façon à libérer l'échantillon biologique 4, sans pour autant faire obstacle à une bonne circulation des billes 3 dans le conteneur 1.

Les billes 3 des différents sous-ensembles 31,32 peuvent être réalisées dans des matériaux identiques ou différents.

Les billes 3 peuvent ainsi être réalisées en matière plastique, en verre, en acier, en céramique, ou tout autre matériau adapté à une utilisation en milieu stérile.

Les billes 3 des différents sous-ensembles peuvent être de diamètres distincts ou identiques.

Le conteneur 1 comprend au moins un sous-ensemble comprenant au moins une bille 3. Le conteneur 1 peut comprendre de l'ordre d'une dizaine de billes, en fonction de sa taille et du diamètre des billes.

Les billes 3 ont un diamètre typiquement compris entre 0.3mm et 50 mm, ou typiquement entre 5mm et 50mm, ou plus précisément entre 10mm et 50mm.

Comme représenté sur la figure 2, le système selon l'invention comprend un secoueur 6 adapté pour entrainer en mouvement le conteneur 1 obturé par le couvercle 2 et contenant les billes 3 ainsi que l'échantillon 4 porté par un support 5.

Dans l'exemple représenté sur les figures, le secoueur 6 comprend une portion de préhension 61 montée sur une base 62.

Comme illustré dans l'exemple de la figure 2, la portion de préhension 61 peut par exemple être réalisée par deux mâchoires antagonistes enserrant d'une part le couvercle 2 et d'autre part le conteneur 1. Une telle configuration permet par exemple de réduire davantage les risques d'ouverture du couvercle 2.

Naturellement, d'autres solutions peuvent être prévues, par exemple une portion de préhension 61 enserrant le conteneur 1, ou tout autre moyen adapté permettant d'immobiliser le conteneur 1 par rapport à la portion de préhension 61.

De façon illustrative et sans perte de généralité, le conteneur 1 et le secoueur 6 seront décrits dans un système d'axes orthogonaux X,Y,Z, dans lequel les directions X et Y sont des directions horizontales et la direction Z est une direction verticale au sens de la direction de la gravité.

De façon illustrative et sans perte de généralité, le système d'axes X,Y,Z est associé à la base 62.

L'entraînement en mouvement du conteneur 1 est réalisé par le secoueur 6 par l'intermédiaire de la portion de préhension 61.

Le mouvement du conteneur 1 décrit ci-après est le mouvement du vecteur n appliqué au point G, centre de gravité de l'ensemble formé par le conteneur 1 vide et le couvercle 2 fixé au conteneur 1, dans lequel la direction du vecteur n est une direction fixe dans un repère propre au conteneur 1.

Autrement dit, le vecteur n subit les mouvements de rotation et de translation appliqués au conteneur 1 par le secoueur 6.

Le mouvement du conteneur 1 provoqué par le secoueur 6 entraine une mise en mouvement de son contenu, à savoir les billes 3, l'échantillon 4, le liquide 7, ainsi que le support 5 le cas échéant. Les billes 3, l'échantillon 4, le liquide 7 et le support 5 sont libres de se déplacer à l'intérieur du conteneur 1 obturé par le couvercle 2, de sorte que les mouvements des billes 3, de l'échantillon 4, du liquide 7 et du support 5 sont de nature plus complexe que le mouvement du conteneur 1.

Ce mouvement provoque des chocs des billes 3 sur le support 5 portant l'échantillon 4, ainsi qu'un mélange du contenu du conteneur 1.

Les chocs des billes 3 sur le support 5 entraînent sa fragmentation, par exemple par broyage, facilitant la libération de l'échantillon 4.

Les chocs des billes 3 sur l'échantillon 4 entrainent sa séparation du support 5, tandis que le mélange du contenu du conteneur 1 permet de réaliser une homogénéisation de ce contenu.

En particulier, les dimensions des billes utilisées permettent de réduire voire de prévenir une dégradation à l'échelle cellulaire des échantillons biologiques, notamment par lyse des membranes cellulaires, ce qui autorise par exemple le recueillement de cellules bactériennes viables, dont la culture et l'analyse sont alors possibles.

Le secoueur 6 est avantageusement configuré de manière à entrainer le conteneur 1 selon un mouvement non rotatif curviligne. Ce mouvement permet en effet d'éviter qu'un effet centrifuge ne se produise dans le conteneur 1, ce qui nuit notamment à l'homogénéisation de l'échantillon car les éléments contenus dans le conteneur 1 ont alors tendance à se plaquer contre les parois du conteneur 1.

Le mouvement appliqué au conteneur 1 par le secoueur 6 est par exemple un mouvement non plan.

Le mouvement appliqué au conteneur 1 par le secoueur 6 peut par exemple être une combinaison de plusieurs mouvements distincts, appliqués simultanément ou successivement. Le mouvement peut par exemple comprendre un mouvement de rotation selon un ou plusieurs axes, un mouvement de va et vient, par exemple en translation et/ou en rotation. Il est en effet compris qu'une composition de mouvements comprenant un mouvement de rotation peut être un mouvement résultant non-rotatif. Le mouvement est adapté pour entrainer des chocs entre les billes 3 et l'échantillon. Le mouvement n'est ainsi typiquement pas un mouvement de rotation uniforme du type centrifugation, qui aurait tendance à former un mouvement d'ensemble de rotation des différents éléments contenus dans le conteneur 1, lequel mouvement d'ensemble entraînerait peu de chocs avec l'échantillon.

Par exemple, ledit mouvement peut être une succession de mouvements rectilignes (translations) alternatifs, c'est-à-dire de va-et-vient selon une même direction, lesdits mouvements rectilignes successifs ayant des directions successives deux à deux formant un angle supérieur à 2°, supérieur à 4°, supérieur à 10°, supérieur à 30°, voire supérieur à 50°. Par exemple, ledit mouvement peut être une succession de deux, trois, quatre ou plus mouvements rectilignes alternatifs. Le cas échéant, les directions des mouvements rectilignes successifs de trois mouvements rectilignes successifs peuvent par exemple être contenues dans un plan commun ou non contenues dans un plan commun.

Une succession de mouvements avec des directions formant de tels angles permet de prévenir et/ou d'interrompre des effets centrifuges du liquide 7 et/ou des billes 3 au sein du conteneur 1.

Par exemple, ledit plan commun est sensiblement parallèle avec le couvercle 2 lorsque le couvercle 2 est assemblé avec le conteneur 1 pour assurer sa fonction d'obturation étanche. Cela permet notamment de réduire la pression qu'exerce le liquide 7 et/ou les billes 3 sur le couvercle 2, et de réduire les risques de perte d'étanchéité par endommagement du couvercle.

Selon un autre exemple, ledit mouvement peut être la combinaison d'une succession de mouvements dans un plan de référence, par exemple rotation(s) et/ou de translation(s), et d'un mouvement de bascule dudit plan de référence. Ladite succession de mouvements dans un plan peut être comprise dans un disque de diamètre inférieur à une longueur R, inférieure à une longueur égale à 0.8*R, voire inférieure à une longueur égale à 0.6*R, où la longueur R en mm est égale à la racine cubique du volume intérieur du conteneur 1 obturé du couvercle 2 exprimé en mm³.

Le mouvement de bascule dudit plan de référence peut par exemple être un mouvement de bascule d'inclinaison angulaire ne dépassant pas 10°, ne dépassant pas 7°, voire ne dépassant pas 4°. Autrement dit, le vecteur normal au dit plan de référence est contenu dans un cône de révolution de demi-angle au sommet ne dépassant pas 10°, ne dépassant pas 7°, voire ne dépassant pas 4°.

L'utilisation de plusieurs sous-ensembles de billes 3 ayant des diamètres et/ou réalisées dans des matériaux distincts permet de rendre plus aléatoire le mouvement des billes 3, entrainant des chocs plus nombreux et notamment entre les billes 3, ce qui renforce l'effet d'homogénéisation.

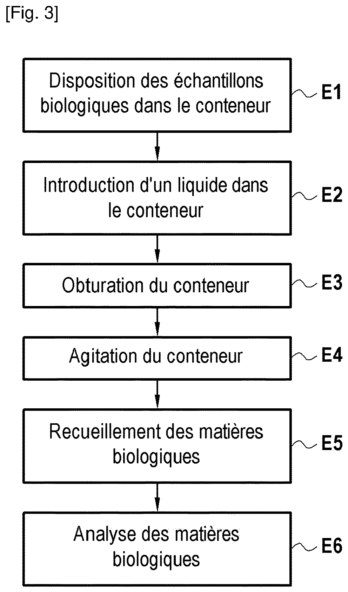

La figure 3 représente schématiquement un procédé de préparation d'échantillon selon un aspect de l'invention.

Lors d'une première étape E1, on dispose un échantillon 4 dans un conteneur 1, le conteneur comprenant des billes 3.

L'échantillon 4 est typiquement un échantillon biologique, par exemple d'origine microbienne, par exemple des bactéries ou des champignons.

L'échantillon 4 peut éventuellement être attaché à un support 5 dont on souhaite le détacher.

Par exemple, l'échantillon 4 peut être un biofilm.

Le support 5 peut comprendre une partie de support d'origine biologique et/ou une partie de support d'origine non biologique.

La partie de support d'origine biologique peut par exemple comprendre des cellules d'origine non végétale, en particulier d'origine animale, plus particulièrement d'origine humaine.

La partie de support d'origine non biologique peut par exemple comprendre une vis à os ou de tissu ostéo-articulaire, typiquement en métal.

Typiquement, la partie de support d'origine biologique est portée par la partie de support d'origine non biologique, et un biofilm que l'on souhaite caractériser est présent entre les deux parties de support.

Dans le cas d'une greffe, par exemple, on peut chercher à caractériser la population microbienne d'un biofilm formé entre une vis implantée et les tissus du patient.

Lors d'une seconde étape E2 optionnelle, on introduit dans le conteneur un liquide, typiquement un liquide de conservation, dans le cas où le dispositif n'en présenterait pas initialement ou dans le cas où l'échantillon 4 ne serait pas sous forme liquide.

Lors d'une troisième étape E3, on obture le conteneur 1 de manière étanche au moyen d'un couvercle 2.

Lors d'une quatrième étape E4, on agite ensuite le conteneur 1 au moyen d'un secoueur 6, de manière à mettre en mouvement le conteneur 1 et ainsi à mettre en mouvement son contenu, de sorte que les billes 3 provoquent des chocs sur l'échantillon, de manière à le séparer d'un support 5 sur lequel il est éventuellement disposé, à le fragmenter, et à réaliser un mélange homogène.

Plusieurs étapes d'agitation successives peuvent être réalisées si nécessaire, au moyen d'actionneurs distincts réalisant par exemple des mouvements distincts.

Le récipient 1 peut ainsi être fourni à un utilisateur pré-rempli de billes 3 et d'un liquide 7, ou pré-rempli uniquement de billes 3 par exemple dans le cas où l'utilisateur souhaite préparer un échantillon déjà sous forme liquide.

Le conteneur 1 est fourni à l'état stérile, obturé de manière étanche. L'utilisateur peut alors l'ouvrir pour y disposer l'échantillon 1, et procéder comme indiqué précédemment en références aux étapes E1 à E4.

Le liquide 7 peut être un liquide de conservation. Par liquide de conservation, on entend un liquide permettant de garder viables les microorganismes sans pour autant favoriser leur croissance, par exemple du sérum physiologique, de l'eau stérile ou de l'eau purifiée.

Un tel liquide de conservation est par exemple adapté pour une utilisation avec des échantillons biologiques solides ou semi-solides tels que des prélèvements de tissus afin de disperser les matières biologiques en vue des analyses.

Lors d'une cinquième étape E5 optionnelle, la matière biologique résiduelle isolée du support 5 peut être recueillie. Par exemple, une partie surnageante de la matière homogénéisée peut être recueillie.

Les étapes E1 à E5 telles que décrites ci-avant permettent de maintenir vivant l'échantillon 4 ou une partie de l'échantillon 4, notamment dans des conditions permettant d'en effectuer l'analyse.

Le recueillement de la matière biologique résiduelle peut comprendre la disposition de matière biologique résiduelle dans un milieu de culture.

Le milieu de culture peut par exemple être un milieu de culture sélectif, c'est-à-dire un milieu de culture favorisant la croissance de certains microorganismes et/ou prévenant la croissance de certains microorganismes.

Par microorganismes, on entend par exemple des bactéries, typiquement pathogènes.

Le recueillement de microorganismes faisant partie de la matière biologique résiduelle permet alors de réaliser leur analyse lors d'une sixième étape E6 optionnelle.

Par analyser les microorganismes, on entend par exemple déterminer au moins un niveau de classification taxonomique d'un organisme.

L'analyse des organismes peut être réalisée au moyen d'outils d'analyse courants du domaine de la microbiologie.

Dans le cas d'espèce, la présence de microorganismes, éventuellement pathogènes, et leur nature peut alors être identifiée sur des échantillons biologiques.

Dans le cas d'un échantillon biologique provenant d'un patient, ces résultats d'analyse pourront dans un second temps être soumis à l'expertise d'un professionnel de la santé qui pourra alors éventuellement constater une présence anormale d'agents pathogènes et ainsi réaliser un diagnostic, par exemple d'une infection bactérienne.

## Revendications

1. Procédé de préparation d'échantillons biologiques pour une analyse bactériologique, dans lequel
- on dispose un support (5) portant un échantillon (4) biologique dans un conteneur (1) contenant des billes (3),
- on obture le conteneur (1) de manière étanche, **caractérisé en ce que**
- on agite le conteneur (1) dans lequel sont disposés l'échantillon (4), les billes (3), de manière à mettre en mouvement les billes (3) et ainsi provoquer des chocs sur le support (5) de manière à séparer l'échantillon (4) du support (5).

2. Procédé selon la revendication 1, dans lequel on prélève et on dispose un résidu de l'échantillon (4) isolé du support (5) dans un milieu de culture, optionnellement un milieu de culture sélectif.

3. Procédé selon la revendication 2, dans lequel on caractérise la présence ou l'absence de microorganismes pathogènes dans le résidu d'échantillon.

4. Procédé selon l'une quelconque des revendications 1 à 3, dans lequel on agite le conteneur (1) selon un mouvement non rotatif.

5. Procédé selon l'une quelconque des revendications 1 à 3, dans lequel on agite le conteneur (1) selon un mouvement comprenant au moins une combinaison de mouvements parmi les combinaisons suivantes :
- une combinaison de translations alternatives ayant des directions successives deux à deux formant un angle supérieur à 10° ; et
- une combinaison d'au moins une translation et d'au moins une rotation.

6. Procédé selon l'une quelconque des revendications 1 à 5, dans lequel préalablement à l'obturation du conteneur (1), on ajoute un liquide (7) dans le conteneur (1).

7. Procédé selon l'une quelconque des revendications 1 à 6, dans lequel l'échantillon (4) est un biofilm.

## Patentansprüche

1. Verfahren zum Aufbereiten biologischer Proben für eine bakteriologische Analyse, wobei
- ein Träger (5), der eine biologische Probe (4) trägt, in einem Kügelchen (3) enthaltenden Behälter (1) angeordnet wird,
- der Behälter (1) dicht verschlossen wird,
**dadurch gekennzeichnet, dass**
- der Behälter (1), in dem die Probe (4) und die Kügelchen (3) angeordnet sind, geschüttelt wird, so dass die Kügelchen (3) in Bewegung gesetzt werden und somit Stöße auf dem Träger (5) hervorrufen, um die Probe (4) von dem Träger (5) zu trennen.

2. Verfahren nach Anspruch 1, wobei ein von dem Träger (5) isolierter Rest der Probe (4) entnommen und in einem Nährmedium, wahlweise einem selektiven Nährmedium, angeordnet wird.

3. Verfahren nach Anspruch 2, wobei das Vorliegen oder das Fehlen von pathogenen Mikroorganismen in dem Probenrest charakterisiert wird.

4. Verfahren nach einem der Ansprüche 1 bis 3, wobei der Behälter (1) mit einer nicht drehenden Bewegung geschüttelt wird.

5. Verfahren nach einem der Ansprüche 1 bis 3, wobei der Behälter (1) mit einer Bewegung geschüttelt wird, die mindestens eine Kombination von Bewegungen aus den folgenden Kombinationen umfasst:
- einer Kombination von abwechselnden Translationen, die paarweise aufeinanderfolgende Richtungen aufweisen, die einen Winkel größer als 10° bilden; und
- einer Kombination aus mindestens einer Translation und aus mindestens einer Rotation.

6. Verfahren nach einem der Ansprüche 1 bis 5, wobei vor dem Verschließen des Behälters (1) eine Flüssigkeit (7) zu dem Behälter (1) hinzugefügt wird.

7. Verfahren nach einem der Ansprüche 1 bis 6, wobei die Probe (4) ein Biofilm ist.

## Claims

1. A method for preparing biological samples for bacteriological analysis, wherein:
- a support (5) carrying a biological sample (4) is placed in a container (1) containing beads (3),
- the container (1) is sealed in a hermetic manner, **characterized in that**
- the container (1) in which the sample (4) and the beads (3) are disposed is shaken so as to move the beads (3) and thus cause shocks on the support (5) so as to separate the sample (4) from the support (5).

2. The method according to claim 1, wherein a residue of the sample (4) isolated from the support (5) is taken and placed in a culture medium, optionally a selective culture medium.

3. The method according to claim 2, wherein the presence or absence of pathogenic microorganisms in the sample residue is characterized.

4. The method according to any one of claims 1 to 3, wherein the container (1) is shaken with a non-rotational movement.

5. The method according to any one of claims 1 to 3, wherein the container (1) is shaken according to a movement comprising at least one combination of movements from the following combinations:
- a combination of alternative translations having successive directions, two at a time forming an angle greater than 10°; and
- a combination of at least one translation and at least one rotation.

6. The method according to any one of claims 1 to 5, wherein, prior to sealing the container (1), a liquid (7) is added to the container (1).

7. The method according to any one of claims 1 to 6, wherein the sample (4) is a biofilm.
